# EUROPEAN PATENT SPECIFICATION

(11) **EP 0 500 611 B1**
(45) Date of publication and mention of the grant of the patent: **09.10.1996**
(21) Application number: 90916195.2
(22) Date of filing: 02.11.1990
(51) Int. Cl.: G01N 33/543, G01N 33/542, G01N 33/553

(54) **ANALYTICAL DEVICE AND METHODS**
ANALYTISCHE VORRICHTUNG UND VERFAHREN
DISPOSITIF ET METHODES D'ANALYSE

(30) Priority: 04.11.1989 GB 8924951
(43) Date of publication of application: 02.09.1992
(73) Proprietor: FISONS plc, Ipswich Suffolk IP1 1QH (GB)
(72) Inventor: MOLLOY, James, Oscar, Surrey SM2 6BP (GB)
(74) Representative: Jones, Stephen Anthony
(86) International application number: GB9001684
(87) International publication number: WO9106862

(56) References cited:
- EP-A- 0 171 148
- WO-A-88/07202
- WO-A-89/08260
- GB-A- 2 174 802

## Description

This invention relates to apparatus and methods for determining analytes in solution, more particularly to the determination of haptens.

A number of different instrumental techniques have been suggested for the determination of chemical and biochemical analytes in solution. Many of these have involved the immobilisation of a binding partner for the analyte on a surface, eg of glass or metal, and then bringing the solution containing the analyte into contact with the surface. Some change in a property of that surface, eg refractive index, due to binding of the analyte with the immobilised binding partner is then monitored, eg by evanescent wave or surface plasmon resonance techniques. The magnitude of this change gives a qualitative and/or quantitative indication of the presence of analyte in the sample.

Haptens (small molecules capable of being bound by antibody but not necessarily themselves immunogenic) are difficult to determine by these methods since they are, by definition, small molecules, the binding of which causes only a small change in the surface property.

WO 88/07202 describes immobilisation of antigens or antibodies etc onto a layer of an oxide of silicon that is coated on a grating.

GB-A-2174802 describes an optic waveguide biosensor having an organic sensitised coating, light coupling-members, and between these two a light-reflecting and partially transmissive layer of a thickness which is such as to afford light coupling by frustrated total internal reflection.

PCT Patent Application WO 89/08260 describes a method of determining haptens in a sample which involves the use of surface plasmon resonance. In order to carry out such a method, the hapten, or in an alternative method an antibody to the hapten, must be linked to the thin layer of metal in which the surface plasmon resonance occurs. Modifications to this method have been described in PCT Patent Application WO 90/11525.

We have now found that the progress of assay methods analogous to those disclosed in WO 89/08260 and WO 90/11525 may be monitored not by surface plasmon resonance but by using devices based on the phenomenon known as frustrated total reflection. The use of this form of detection has numerous advantages over the use of surface plasmon resonance. Assays carried out using the device in accordance with the invention are particularly useful in the determination of haptens.

The principles of frustrated total reflection (FTR) are well-known; the technique is described, for example, by Bosacchi and Oehrle [Applied Optics (1982), 21, 2167-2173]. An FTR device for use in immunoassay is disclosed in US Patent 4,857,273 and comprises a cavity layer bounded on one side by the sample under investigation and on the other side by a spacer layer which in turn is mounted on a substrate. The substrate-spacer layer interface is irradiated with monochromatic radiation such that total reflection occurs, the associated evanescent field penetrating through the spacer layer. If the thickness of the spacer layer is correct and the incident wave vector matches one of the resonant mode propagation constants, the total reflection is frustrated and radiation is coupled into the cavity layer. The cavity layer must be composed of material which has a higher refractive index than the spacer layer and which is transparent at the wavelength of the incident radiation. These requirements preclude the use of metals for the cavity layer due to the high absorbtivity at visible wavelengths.

According to the invention, therefore, there is provided a biosensor comprising
a) a cavity layer of transparent dielectric material of refractive index n₃ having immobilised upon it a hapten or hapten analogue, the cavity layer having a thickness of from 30 to 150nm,
b) a dielectric substrate of refractive index n₁, and
c) interposed between the cavity layer and the substrate, a dielectric spacer layer of refractive index n₂, with a thickness of 500 to 1500nm
wherein the refractive index n₃ of the cavity layer and the refractive index n₁ of the substrate both exceed the refractive index n₂ of the spacer layer,
the arrangement being such that, when the interface between the substrate and the spacer layer is irradiated with light such that total reflection occurs, the total relection may be frustrated by the propagation of a resonant guided mode within the cavity layer.

The device according to the invention is advantageous primarily in that its use may lead to greater sensitivity in the determination of haptens in solution than is possible using known techniques, eg the technique involving surface plasmon resonance described in PCT Patent Application WO 89/08260. The enhanced sensitivity may be due, for example, to the fact that the propagation distance of the light coupled into the cavity layer may be optimised, eg by appropriate choice of materials and layer thicknesses. In addition, it may be possible, by appropriate choice of device parameters, to confine the evanescent field more closely to the surface of the cavity layer than is possible with corresponding surface plasmon resonance devices (the choice of parameters being made according to the principle described in European Patent No 0075353). These advantages may be of especial significance in the determination of haptens, which are necessarily rather small species compared with other classes of analyte which may be determined by this and similar methods.

The immobilisation of haptens and antibodies thereto on the dielectric cavity layer may also be more easily accomplished than immobilisation of corresponding species on metal layers. Also, the fabrication of the dielectric structure according to the present invention may be simpler and less costly than that of surface plasmon resonance based sensors involving thin layers of metal.

By 'hapten analogue' is meant any substance which competes with the hapten for binding to an antibody to the hapten. The analogue may be near-identical, or even identical, to the hapten itself.

By 'transparent dielectric material' is meant dielectric material which exhibits little or no absorbtivity at the wavelength(s) of the incident radiation.

In this context, 'light' may include not only visible light but also wavelengths above and below this range, eg in the ultra-violet and infra-red.

Resonant propagation of a guided mode in the cavity layer will occur, for a given wavelength, at a particular angle of incidence of the exciting radiation. Thus, two basic measurement approaches are possible: scanning the angle of incidence at fixed wavelength or scanning the wavelength at a fixed angle of incidence. The former approach, using monochromatic radiation, is preferred since it allows the use of a laser source, simplifying the problem of optical collimation, and avoids dispersion effects, thereby simplifying the analysis of the results.

The angular position of the resonant effect depends on various parameters of the multilayer device constituting the present invention, such as the refractive indices and thicknesses of the various layers. In general, it is a pre-requisite that the refractive index n₃ of the cavity layer and the refractive index n₁ of the substrate should both exceed the refractive index n₂ of the spacer layer. Also, since at least one mode must exist in the cavity to achieve resonance, the cavity layer must exceed a certain minimum thickness.

Suitable transparent dielectric materials for the cavity layer include zirconium dioxide, titanium dioxide, silicon dioxide, aluminium oxide and tantalum oxide.

The cavity layer may be prepared by known techniques, eg vacuum evaporation.

The dielectric spacer layer must also be transparent to the incident radiation and must have a lower refractive index than both the cavity layer and the substrate. The layer may, for example, comprise an evaporated or sputtered layer of magnesium fluoride. In this case an infra-red light injection laser may be used as light source. The light from such a source typically has a wavelength of 800nm. For light of lower wavelength it may be necessary to use a different material for the spacer layer, eg aluminium oxide. Other suitable materials include lithium fluoride and silicon dioxide. Apart from the evaporation and sputtering techniques mentioned above, the spacer layer may be deposited on the substrate by a sol-gel process. The latter process is particularly preferred where the spacer layer is of silicon dioxide.

The substrate is typically of glass or quartz, although other materials may be used, eg polymeric materials. In order to couple radiation into and out of the system, it may be necessary to use conventional coupling means, eg grating couplers or prism couplers. It may further be necessary to provide a layer of index-matching fluid or the like between a prism coupler and the substrate.

For ease of fabrication and handling, the substrate may take the form of a glass chip which may be, for example, about lmm thick and 10mm square. For simplicity of use, it may be desirable for the chip to be, or to be part of, a disposable unit.

The refractive index of the substrate (n₁) must be greater than that (n₂) of the spacer layer but the thickness of the substrate is generally not critical to the performance of the invention.

By contrast, the thicknesses of the cavity layer and the spacer layer must be so chosen that resonance occurs within an appropriate range of coupling angles. The spacer layer will typically have a thickness of the order of several hundred nanometres, thus, from 500 to 1500nm, eg 1000nm. The cavity layer typically has a thickness of a few tens of nanometres, thus, 30 to 150nm, eg 100nm.

In the embodiment of the invention, the cavity layer has a thickness of 30 to 150nm and comprises a material selected from zirconium dioxide, titanium dioxide, silicon dioxide, tantalum oxide and aluminium oxide, and the spacer layer has a thickness of 500 to 1500nm and comprises a material selected from magnesium fluoride, lithium fluoride and silicon dioxide, the choice of materials being such that the refractive index of the spacer layer is less than that of the cavity layer.

Preferred materials for the cavity layer and the spacer layer are tantalum oxide and silicon dioxide respectively.

The biosensor device according to the invention will generally form part of an apparatus comprising, in addition, a light source and suitable detection means.

Any convenient source of radiation may be used as the source of the incident light but, as noted above, it is preferable to use monochromatic radiation and the most convenient source of such radiation is a laser. The choice of laser will depend inter alia on the materials used for the various layers and some examples have already been given.

The scanning of the wavelength may be performed either sequentially or simultaneously ie by varying the angle of incidence of a narrow beam of light or by simultaneously irradiating over a range of angles using a fan-shaped beam of light as described (in connection with surface plasmon resonance) in European Patent Application No 0305109A. In the former case, a single-channel detector may be used which is mechanically scanned over a range of angles; in the latter case, in which a range of angles is irradiated simultaneously, it will generally be necessary to use a multi-channel detector.

At resonance, the incident light is coupled into the cavity layer by FTR, propagates a certain distance along the cavity layer, and couples back out (also by FTR). The propagation distance depends on the various device parameters but is typically of the order of 1 or 2mm.

In general, at resonance, the reflected light will undergo a phase change and it may be the angular position at which this phase change occurs which is detected. Changes on the surface of the cavity layer, eg binding of hapten to immobilised antibody, cause changes in the refractive index of the sample and hence shift the angular position of the resonance.

There may also be a reduction in the intensity of the reflected light, eg if the immobilised species are absorbing at the wavelength of the incident radiation. In this case, this reduction in intensity may be used to monitor the resonance.

As mentioned above, the biosensor device according to the invention is particularly useful in methods of assaying biological samples for the presence of haptens.

Thus, in other aspects of the invention there are provided a method of determining a hapten in a sample, which comprises:
a1) contacting, either sequentially or simultaneously, a specific binding partner of the hapten and the sample containing the hapten, with a layer of transparent dielectric material of thickness 30 to 150nm, said layer constituting the resonant cavity of a frustrated total reflection biosensor, the layer of transparent dielectric material having immobilised upon it a hapten or an analogue thereof, or
a2) contacting, either sequentially or simultaneously, a conjugate of the hapten or of an analogue thereof and the sample containing the hapten, with a layer of transparent dielectric material of thickness 30 to 150nm, said layer constituting the resonant cavity of a frustrated total reflection biosensor, the layer of transparent dielectric material having immobilised upon it a specific binding partner of the hapten or analogue,
   and
b) monitoring binding of the specific binding partner to the immobilised hapten or hapten analogue or binding of the hapten conjugate to the immobilised specific binding partner, as the case may be, by means of frustrated total reflection.

In both methods, the immobilised species (hapten, hapten analogue or specific binding partner) may be covalently bound to the transparent dielectric layer by methods which are well known to those skilled in the art. In general, however, in order to facilitate immobilisation, the dielectric layer will be derivatised or activated. The derivatisation or activation of the surface will be such as to provide coupling sites for the species to be immobilised without appreciably affecting the reactivity or affinity of the immobilised species for its specific binding partner.

For example, the dielectric layer may be reacted with a silane-based coupling compound in a known manner. A suitable such reagent is, for example, a terminal amino-alkyl trimethoxysilane, eg the 3-aminopropyl compound, used at a concentration of about 2%w/v in acetone. Details of immobilisation techniques using this reagent have been described by Weetall [see, for example, US Patent 3 652 761 and "Immobilized Biochemicals and Affinity Chromatography", R B Dunlop (Ed), Plenum Press, New York (1974), pp 191-212], along with a description of other silyl compounds and the methods by which carboxyl, amino and other reactive groups of antibody or antigen (or their fragments) may be covalently bound to various inorganic materials.

After reaction with the amino-silane reagent, the amino terminals immobilised on the dielectric layer may in turn be reacted with glutaraldehyde (eg a 2% solution of pH 7), excess reagents removed and the activated surface with immobilised aldehyde groups then treated with a solution of the species to be immobilised, eg an antibody immunoglobulin at a concentration of about 1% w/v. Continuous or near-continuous layers of immobilised species can be obtained with a surface density of about 0.5-2.0 µg/cm². Excess reagents can then be removed by, for example, washing in a strong buffer solution (0.1M acetate, 0.5M NaCl, pH4-5), then neutral buffer washing (pH 7-7.4), followed by pH 9-10 washing and neutralisation, eg with neutral tris buffer.

An alternative method for coupling proteins to the dielectric surface involves the use of epoxy-silane reagents, especially glycidyloxypropyltrimethoxysilane, eg at a concentration of about 2%v/v in toluene for about 2 hours at 70°C as described by Herman et al [J Chromatogr Sci (1981), 19(9), 470-476]. In this method the use of aldehyde reagents is unnecessary, since the epoxysilylated dielectric layer can react directly with the protein.

Methods of immobilising antigens or antibodies to polymeric surfaces are also well-known, and those skilled in the art will recognise the applicability of these methods when the transparent dielectric layer is polymeric. For example, the coupling may be in the form of the substitution of an appropriate radical for a hydrogen on any of the polymer's functional groups.

Particularly in method a1) (in which the immobilised species is the relatively small hapten or hapten analogue), the coupling site may incorporate an intermediate linking group. The use of such linking groups ensures sufficient separation between the surface and the species bound by the immobilised specific binding partners so as to minimize steric hindrance of the specific binding process.

According to a preferred aspect of the invention, therefore, there is provided a layer of transparent dielectric material having immobilised upon it a linking group to which is linked a hapten or hapten analogue.

According to another aspect of the invention, there is provided a method of immobilising a hapten or hapten analogue on a layer of transparent dielectric material, which method comprises linking the hapten to an intermediate linking group to form a conjugate and then immobilising that conjugate on the surface of the layer of transparent dielectric material.

An example of a linking group which may be used is a polyethylene chain, as for example in the case of a 1,6-diaminohexane or 6-aminohexanoic acid bound to the dielectric surface through a peptide bond and respectively providing a free primary amino and a free carboxyl group for covalently binding to the carboxyl or amino termination of a protein. These coupling materials both provide a 6-carbon chain between terminations, thereby spacing the immobilised species from the surface by the corresponding distance. Similar appropriate coupling and intermediate linking groups are well known in the fields of immunoassay and affinity chromatography.

The linking group is preferably part of a macromolecular coating of such units which substantially completely covers that portion of the surface of the layer of transparent dielectric material used for the analysis. The linking group is preferably a protein which is chemically, eg covalently, linked to the hapten, eg by a suitable bridge structure. Techniques by which this linkage may be achieved will be apparent to those skilled in the art. One example is the mixed anhydride technique. Suitable hapten-bridge-protein conjugates include phenytoin-glucuronide-gamma globulin and phenytoin-glucuronyl-lysozyme.

In method a1), the specific binding partner which is reversibly bound to the immobilised hapten or hapten analogue may simply be an antibody to the hapten or a fragment thereof.

Alternatively, the specific binding partner may be a conjugate of such an antibody or antibody fragment. In this case, the conjugate of the antibody is preferably a conjugate with some substance which is of sufficiently high molecular weight to produce a significant change in the frustrated total reflection characteristics of the device as an indicator of binding of the analyte. The conjugate may be a conjugate with any suitable material to which the antibody can be conveniently covalently bound by a coupling reagent, but is typically a conjugate of the antibody with a macromolecule such as a protein, or with a high refractive index particle such as a polystyrene bead or a colloidal metal. Macromolecules which may be employed include bovine serum albumin, human serum albumin, egg albumin and polylysine, as well as species such as immunoglobulin, lipid A, collagen and the like. A coupling reagent is normally used to effect the covalent binding between the antibody and the macromolecule. In addition to the linking groups mentioned above, such coupling reagents include hydrazides, azides, cyanogen bromide, N,N-o-phenyldimaleimide, m-maleimidobenzoyl-N-hydroxysuccinimide ester and the like. The choice of coupling agent will of course depend on the particular antibody and macromolecule concerned.

In bringing about the covalent binding between the antibody and macromolecule, the coupling reagent is normally added to a solution or mixture of the antibody and/or macromolecule, the concentration of the coupling reagent varying with the particular system involved. After incubation, typically for several hours, the antibody is added and the mixture further incubated. The conjugate is then isolated using known techniques.

The conjugate is then typically diluted in a suitable buffer, such as carbonate-bicarbonate, to a concentration which will generally be in the range of 10 to 200 ng/ml, eg about 100ng/ml. Other buffers can of course be used, eg glycine buffer pH 9.5, and other dilutions, eg 50ng/ml. The solution is then contacted with the transparent dielectric layer having immobilised upon it the hapten or hapten analogue. Contact is maintained for a sufficient time to permit the conjugate to bind to the immobilised species. After binding is complete, the dielectric surface is washed free of unbound conjugate. This washing can be effected using any suitable solution, eg phosphate-buffered saline, or distilled water. Several washings may be necessary to ensure that no unbound conjugate is left in contact with the surface which can then be dried, eg by air drying or freeze-drying, and stored for future use.

In method a2), the hapten conjugate may be prepared by similar methods to those described above. For example, in the case where gentamicin is coupled to bovine serum albumin by means of glutaraldehyde, the coupling reagent is initially mixed with the macromolecule to provide a concentration of coupling reagent to macromolecule of about 2:1 by weight, whereupon after a 3-hour incubation period at room temperature, the solution is chromatographed on a column of cross-linked dextran (with a molecular weight exclusive limit of 2500) and the first 20ml after the void volume are collected. To the resulting collected mixture is added 200mg of hapten, whereupon the resulting mixture is incubated for about 20 hours at room temperature with stirring, and then 500mg of glycine is added. The final mixture is incubated an additional two hours at room temperature, after which this conjugate solution is dialyzed for about three days against distilled water at room temperature and then lyophilized to provide a finished macromolecule-hapten conjugate.

The immobilised specific binding partner in method a2) may be an antibody to the hapten or hapten analogue, or a fragment thereof. Immobilisation may be effected by similar means to those described above.

In both methods the order of addition of the reagents is not critical. In method al), however, it is preferred that the specific binding partner is first added to the immobilised hapten or analogue, which is then washed to remove any unbound material. Then the sample is brought into contact with the layer of transparent dielectric material, on which the specific binding partner has previously been reversibly bound to the immobilised hapten or analogue. In method a2), conjugate is first reversibly bound to the immobilised specific binding partner, and the sample thereafter brought into contact with the surface bearing the immobilised species.

In a modification of method a2), the hapten or hapten analogue is conjugated with a species which is a specific binding partner of a further reagent unreactive with the hapten or hapten analogue, or with the immobilised specific binding partner of the hapten or hapten analogue. In this case, the method involves an additional step, viz the addition of the further reagent.

The further reagent may be a specific binding partner to the species to which the hapten is conjugated or, for greater sensitivity, a conjugate of such a specific binding partner, eg with a large macromolecule.

Examples of specific binding systems which may be involved in binding of the further reagent and hapten conjugate are biotin-avidin and biotin-streptavidin.

In a further modification, the further reagent may be a conjugate of a specific binding partner of the species to which the hapten is conjugated with an enzyme capable of catalysing the production of a detectable reaction product at or near the dielectric surface. Examples of suitable enzymes are:
i) Peroxidase, with H₂O₂ and diamino-benzidine as substrates.
ii) Certain oxidoreductases with NAD(P)H and a tetrazolium salt as substrate.
iii) Certain catalase enzymes which are known to generate gas, bubbles of which can be retained close to the dielectric surface.

The enzyme substrate is generally contacted with the surface after the further reagent (containing the enzyme) has been immobilised by complexation at the surface and excess further reagent removed, eg by washing.

Another method of immunoassay in which the present invention may be used involves determining the presence in a sample of an antibody to a particular hapten by contacting immobilised hapten with the sample and monitoring binding of the immobilised hapten with the antibody. This method is, however, of little importance in practice.

Haptens assayed using the device and method of the present invention may be any one or more of a wide variety of substances, eg drugs, animal and plant hormones, antibiotics, pesticides, etc. Examples of hormones which may be assayed include thyroid stimulating hormone, luteinizing hormone, human chorionic gonadotrophin, follicle stimulating hormone, insulin, prolactin, steroid hormones (eg cortisol, estradiol, progesterone, and testosterone), thyroxine and triiodothyronine.

The invention will now be described, by way of illustration only, with reference to the accompanying drawings in which
Figure 1 shows a biosensor device according to the invention,
Figure 2 shows in schematic form a first method of assaying haptens using the device of Figure 1, and
Figure 3 shows in schematic form a second method of assaying haptens using the device of Figure 1.

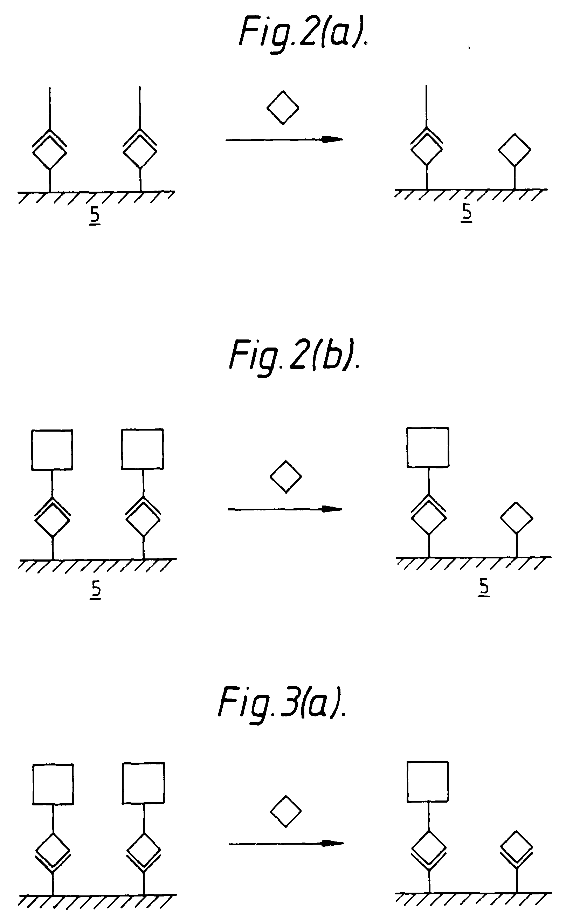

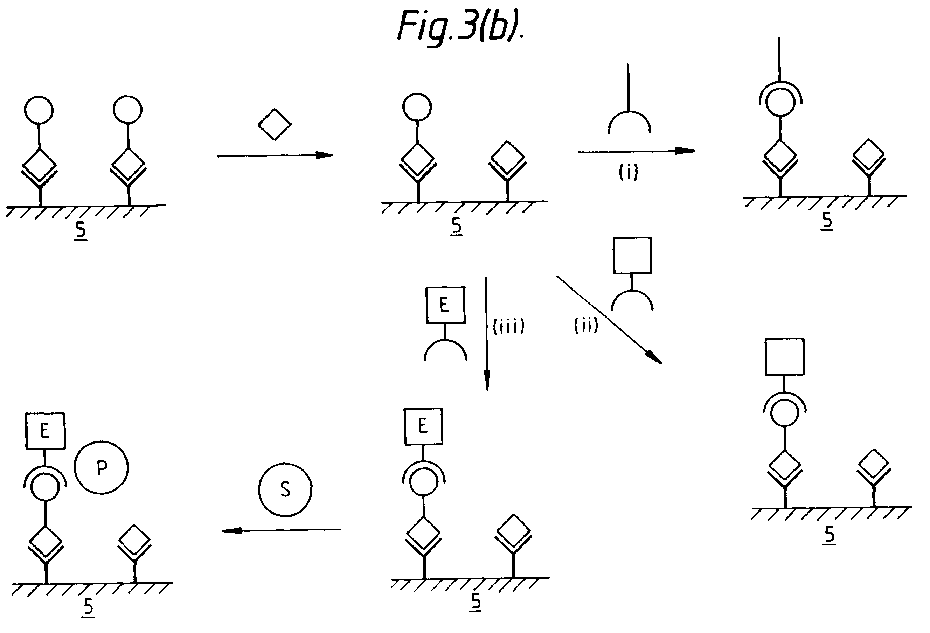

In Figures 2 and 3, the following symbols have these meanings:

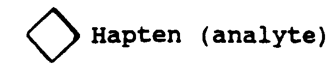

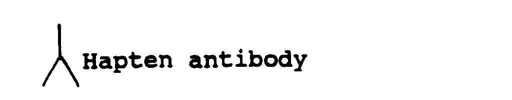

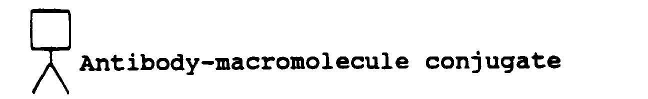

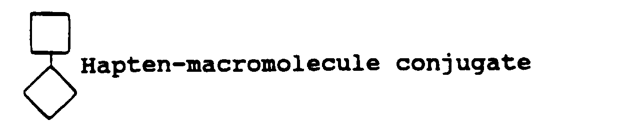

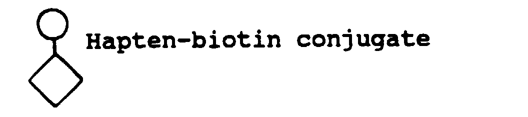

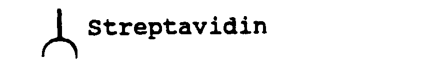

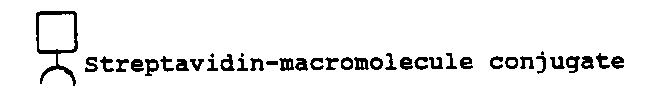

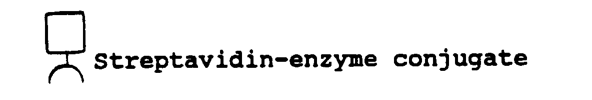

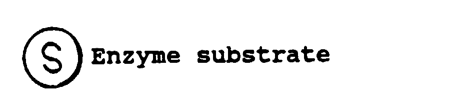

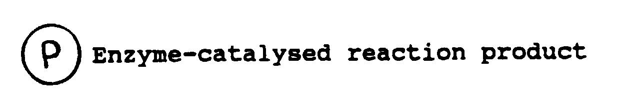

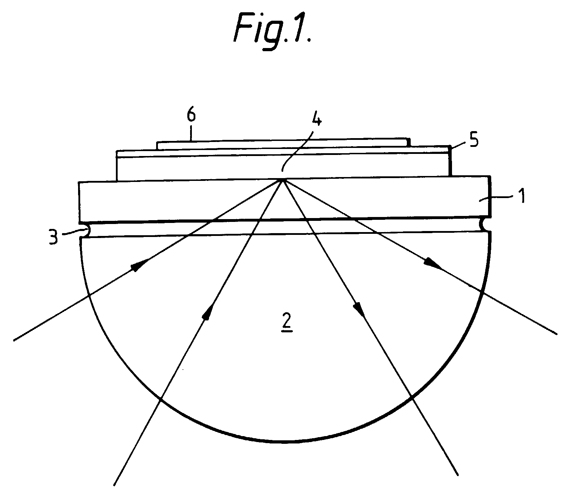

Referring first to Figure 1, a biosensor device employing the principle of frustrated total reflection comprises a glass chip (1) approximately 10mm square and having a thickness of approximately 1mm. The glass chip (1) is mounted on the flat surface of a hemispherical prism (2), a layer of index-matching fluid (3) being interposed between the chip (1) and the prism (2).

A spacer layer (4) of aluminium oxide is formed on the upper surface of the glass chip (1) and has a thickness of approximately 700nm. Formed on the spacer layer (4) is a second layer (5) of zirconium dioxide (approximate thickness 40nm). The lamellar structure is completed by a layer of immobilised biochemical species (6), the nature of which depends on the assay techniqe to be performed and is more closely described below.

In use, the interface between the glass chip (1) and the spacer layer (4) is irradiated with a beam of monochromatic radiation from a laser (not shown). Suitable optics (not shown) are provided to form the incident beam into a fan-shape so that irradiation occurs simultaneously over a range of angles.

Total reflection of the incident beam occurs but, at a particular angle of incidence, the reflection is frustrated by resonant coupling of radiation into the second layer (5), which has a higher refractive index than either of the layers (4,6) above and below it and therefore acts as a resonant cavity. At this angle the reflected radiation undergoes a phase change.

The phase of the reflected radiation is monitored as a function of angle by a suitable detector (not shown) and the angle at which resonance occurs determined. After a sample containing the analyte under test is contacted with the layer of immobilised biochemical species (6), the refractive index of that layer is altered, resulting in a change in the angular position of the resonance. This change is monitored, its occurrence and magnitude providing a qualitative and quantitative indication of the presence of analyte in the sample.

Figure 2 shows a first method of assaying for haptens which may be performed using the apparatus described above. In a first embodiment, shown in Figure 2(a), the surface of the second layer (5) is coated with immobilised linking groups to which molecules of the hapten under test are covalently linked. In a preliminary step, antibodies to the hapten are applied to the surface such that complexation occurs over substantially the whole surface. The layer (6) of immobilised biochemicals thus comprises a layer of hapten-antibody complex immobilised via the linking groups.

When a sample containing the hapten under test (the analyte) is contacted with the active surface (6), the analyte molecules compete with the immobilised haptens for binding of the antibody molecules, with the result that some of the antibodies are liberated from the surface of the device. This causes a change in the refractive index in the vicinity of the surface (due, for example, to the different sizes of the hapten and antibody molecules), altering the angular position of the resonance.

In a second embodiment, shown in Figure 2(b), the effect of addition of analyte is amplified by virtue of the fact that the antibody which is pre-complexed with the immobilised hapten is conjugated with a large macromolecule. This causes a greater change in the local refractive index when analyte hapten competes with immobilised hapten for binding of the antibody.

A second method of assay employing the device of Figure 1 is shown in Figure 3. Antibodies to the analyte hapten are immobilised on the surface of the device and are pre-complexed with hapten conjugates. In a first embodiment, shown in Figure 3(a), the hapten molecules are covalently linked to large protein molecules. The layer of immobilised chemicals (6) thus comprises a layer of immobilised antibody - hapten conjugate complex. Addition of a sample containing analyte haptens results in displacement of some of the hapten conjugates from the surface, again producing a change in refractive index and shift of the resonance.

In the embodiment of Figure 3(b) the complexed hapten is conjugated with one member of a different specific binding pair, in this case biotin. After, or simultaneously with, treatment of the surface with the sample (containing the analyte) a further reagent is added. This further reagent may be:
i) streptavidin, in which case the final complex is immobilised antibody-(hapten-biotin)-streptavidin, or
ii) a streptavidin-macromolecule conjugate, or
iii) a streptavidin-enzyme conjugate.

In the last case, subsequent treatment with enzyme substrate gives rise to a reaction product in the vicinity of the dielectric surface, the enzyme-substrate surface being chosen such that the reaction product produces a substantial change in refractive index in that vicinity.

## Claims

1. A biosensor comprising
a) a cavity layer of transparent dielectric material of refractive index n₃ having immobilised upon it a hapten or hapten analogue, the cavity layer having a thickness of from 30 to 150nm
b) a dielectric substrate of refractive index n₁, and
c) interposed between the cavity layer and the substrate, a dielectric spacer layer of refractive index n₂ with a thickness of 500 to 1500nm
wherein the refractive index n₃ of the cavity layer and the refractive index n₁ of the substrate both exceed the refractive index n₂ of the spacer layer,
the arrangement being such that, when the interface between the substrate and the spacer layer is irradiated with light such that total reflection occurs, the total reflection may be frustrated by the propagation of a resonant guided mode within the cavity layer.

2. A biosensor according to Claim 1, wherein the cavity layer is of a transparent dielectric material selected from the group consisting of zirconium dioxide, titanium dioxide, silicon dioxide, aluminium oxide and tantalum oxide.

3. A biosensor according to Claim 1 or Claim 2, wherein the cavity layer is of tantalum oxide.

4. A biosensor according to any one of Claims 1 to 3, wherein the spacer layer is of silicon dioxide.

5. An apparatus comprising
a) a biosensor according to any one of Claims 1 to 4,
b) a light source, and
c) light detection means.

6. An apparatus according to Claim 5, wherein the light source is a laser.

7. A method of determining a hapten in a sample, which comprises:
a1) contacting, either sequentially or simultaneously, a specific binding partner of the hapten and the sample containing the hapten, with a layer of transparent dielectric material of thickness 30 to 150nm, said layer constituting the resonant cavity of a frustrated total reflection biosensor, the layer of transparent dielectric material having immobilised upon it a hapten or an analogue thereof, or
a2) contacting, either sequentially or simultaneously, a conjugate of the hapten or of an analogue thereof and the sample containing the hapten, with a layer of transparent dielectric material of thickness 30 to 150nm, said layer constituting the resonant cavity of a frustrated total reflection biosensor, the layer of transparent dielectric material having immobilised upon it a specific binding partner of the hapten or analogue,
and
b) monitoring binding of the specific binding partner to the immobilised hapten or hapten analogue or binding of the hapten conjugate to the immobilised specific binding partner, as the case may be, by means of frustrated total reflection.

8. A method according to Claim 7, wherein step a1) comprises the steps of
i) adding the specific binding partner to the immobilised hapten or analogue,
ii) washing to remove any unbound material, and
iii) bringing the sample into contact with the layer of transparent dielectric material, on which the specific binding partner has been reversibly bound to the immobilised hapten or analogue.

9. A method according to Claim 7, wherein the immobilised specific binding partner in step a2) is an antibody to the hapten or hapten analogue, or a fragment thereof.

10. A method according to Claim 7, wherein step a2) comprises the steps of
i) reversibly binding the conjugate to the immobilised specific binding partner, and
ii) bringing the sample into contact with the surface bearing the immobilised species.

11. A method according to Claim 7, wherein the hapten or hapten analogue in step a2) is conjugated with a species which is a specific binding partner of a further reagent unreactive with the hapten or hapten analogue, or with the immobilised specific binding partner of the hapten or hapten analogue, and the method involves, as an additional step, the addition of the further reagent.

12. A method according to Claim 11, wherein the further reagent is a conjugate of the specific binding partner to the species to which the hapten is conjugated.

13. A method according to Claim 12, wherein the further reagent is a conjugate of the specific binding partner with an enzyme capable of catalysing the production of a detectable reaction product at or near the dielectric surface.

## Patentansprüche

1. Ein Biosensor der folgendes umfasst:
a) eine Hohlraumschicht aus durchlässigem dielektrischem Material mit Brechungsindex n₃, die darauf ein Hapten oder Haptenanalogon immobilisiert hat, wobei die Hohlraumschicht eine Dicke von 30 bis 150nm hat,
b) einen dielektrischen Träger mit Brechungsindex n₁, und
c) eine zwischen der Hohlraumschicht und dem Träger zwischengelagerte dielektrische Abstandshalterschicht mit Brechungsindex n₂ mit einer Dicke von 500 bis 1500nm
in dem der Brechungsindex n₃ der Hohlraumschicht und der Brechungsindex n₁ des Trägers beide den Brechungsindex n₂ der Abstandshalterschicht Überschreiten,
in dem die Anordnung so ist, daß, wenn die Grenzfläche zwischen dem Träger und der Abstandshalterschicht mit Licht bestrahlt wird, so daß Totalreflexion stattfindet, die Totalreflexion durch die Fortpflanzung einer resonant gesteuerten Betriebsart in der Hohlraumschicht gestört sein kann.

2. Biosensor nach Anspruch 1, in dem die Hohlraumschicht aus einem durchlässigen dielektrischen Material ist, das von der Gruppe ausgewählt ist, die aus Zirconiumdioxid, Titandioxid, Siliciumdioxid, Aluminiumoxid und Tantaloxid besteht.

3. Biosensor nach Anspruch 1 oder Anspruch 2, in dem die Hohlraumschicht aus Tantaloxid ist.

4. Biosensor nach einem der Ansprüche 1 bis 3, in dem die Abstandshalterschicht aus Siliciumdioxid ist.

5. Eine Vorrichtung, die folgendes umfasst:
a) einen Biosensor nach einem der Ansprüche 1 bis 4,
b) eine Lichtquelle, und
c) ein Lichtauffindungsmittel.

6. Vorrichtung nach Anspruch 5, in der die Lichtquelle ein Laser ist.

7. Verfahren zur Bestimmung eines Haptens in einer Probe, das folgendes umfasst:
a1) man berührt entweder nacheinander oder gleichzeitig einen spezifischen Bindepartner des Haptens und der Probe, die das Hapten enthält, mit einer Schicht aus durchlässigem dielektrischem Material einer Dicke von 30 bis 150nm, wobei die Schicht den resonanten Hohlraum eines Biosensors mit gestörter Totalreflexion bildet, die Schicht aus durchlässigem dielektrischem Material darauf ein Hapten oder ein Analogon davon immobilisiert hat, oder
a2) man berührt entweder nacheinander oder gleichzeitig ein Konjugat des Haptens oder eines Analogons davon und die Probe, die das Hapten enthält, mit einer Schicht aus durchlässigem dielektrischem Material einer Dicke von 30 bis 150nm, wobei die Schicht den resonanten Hohlraum eines Biosensors mit gestörter Totalreflexion bildet, die Schicht aus durchlässigem dielektrischem Material darauf einen spezifischen Bindepartner des Haptens oder des Analogons immobilisiert hat,
und
b) man überwacht, je nach Fall, die Bindung des spezifischen Bindepartners an das immobilisierte Hapten oder Haptenanalogon oder die Bindung des Haptenkonjugats an den immobilisierten spezifischen Bindepartner durch gestörte Totalreflexion.

8. Verfahren nach Anspruch 7, in dem Schritt a1) die folgenden Schritte umfasst:
i) man gibt den spezifischen Bindepartner dem immobilisierten Hapten oder Haptenanalogon zu,
ii) man wäscht, um irgendein ungebundenes Material zu entfernen, und
iii) man bringt die Probe mit einer Schicht aus durchlässigem dielektrischen Material in Berührung, auf welches der spezifische Bindepartner umkehrbar an das immobilisierte Hapten oder Analogon gebunden worden ist.

9. Verfahren nach Anspruch 7, in dem der immobilisierte spezifische Bindepartner in Schritt a2) ein Antikörper zum Hapten oder Haptenanalogon ist, oder ein Bruchteil davon.

10. Verfahren nach Anspruch 7, in dem Schritt a2) die folgenden Schritte umfasst:
i) das Konjugat wird umkehrbar an den immobilisierten spezifischen Bindepartner gebunden,
ii) die Probe wird mit der Oberfläche in Berührung gebracht, die die immobilisierte Art trägt.

11. Verfahren nach Anspruch 7, in dem das Hapten oder das Haptenanalogon in Schritt a2) mit einer Art konjugiert wird, die ein spezifischer Bindepartner eines weiteren Prüfstoffs ist, der nicht mit dem Hapten oder dem Haptenanalogon oder mit dem immobilisierten spezifischen Bindepartner des Haptens oder des Haptenanalogons reagiert, und das Verfahren als zusätzlichen Schritt die Zugabe des weiteren Prüfstoffs einschließt.

12. Verfahren nach Anspruch 11, in dem der weitere Prüfstoff ein Konjugat des spezifischen Bindepartners zu der Art ist, an die das Hapten konjugiert ist.

13. Verfahren nach Anspruch 12, in dem der weitere Prüfstoff ein Konjugat des spezifischen Bindepartners mit einem Enzym ist, das die Herstellung eines auffindbaren Reaktionsprodukts an oder in der Nähe der dielektrischen Oberfläche katalysieren kann.

## Revendications

1. Un biocapteur comportant
a) une couche creuse de matière diélectrique transparente à indice de réfraction n₃ portant un haptène ou analogue d'haptène, la couche creuse ayant une épaisseur de 30 p 150 nm
b) un substrat diélectrique à indice de réfraction n₁, et
c) interposée entre la couche creuse et le substrat, une couche diélectrique d'interposition à indice de réfraction n₂ d'une épaisseur entre 500 et 1500nm
dont l'indice de réfraction n₃ de la couche creuse et l'indice de réfraction n₁ du substrat sont tous deux supérieurs à l'indice de réfraction n₂ de la couche interposée,
l'agencement étant tel que lorsque l'interface entre le substrat et la couche interposée est irradiée de lumière une réflection globale est assurée, ladite réflection globale pouvant être frustrée par la propagation d'un mode résonant guidé dans la couche creuse.

2. Biocapteur selon la revendication 1, dont la couche creuse est formée de matière diélectrique transparente sélectionnée à partir du groupe qui consiste de dioxyde de zircone, dioxyde de titane, dioxyde de silicium, oxyde d'aluminium et oxyde de tantale.

3. Biocapteur selon la revendication 1 ou la revendication 2, dont la couche creuse consiste d'oxyde de tantale.

4. Biocapteur selon l'une ou l'autre des revendications 1 à 3, dont la couche creuse consiste d'oxyde de silicium.

5. Appareil comportant
a) un biocapteur selon l'une ou l'autre des revendications 1 à 4,
b) une source de lumière, et
c) un moyen détecteur de lumière.

6. Appareil selon la revendication 5, dont la source de lumière est un laser.

7. Méthode de détermination d'un haptène dans une éprouvette, comportant:
a1) la mise en contact soit en séquence soit simultanément, d'un élément spécifique de liaison de l'haptène et de l'éprouvette comportant ledit haptène, avec une couche de matière diélectrique transparente d'une épaisseur de 30 à 150 nm, ladite couche formant le creux résonant d'un biocapteur de réflection globale frustrée, la couche de matière diélectrique transparente portant immobilisé un haptène ou analogue d'haptène, ou
a2) la mise en contact soit en séquence soit simultanément, d'un conjugué d'haptène ou de son analogue et l'éprouvette portant l'haptène, avec une couche de matière diélectrique transparente d'une épaisseur de 30 à 150 nm, ladite couche formant le creux résonant d'un biocapteur de réflection globale frustrée, la couche de matière diélectrique transparente portant immobilisé un élément de liaison d'haptène ou analogue,
et
b) la surveillance de la liaison de l'élément spécifique de liaison avec en immobilisation l'haptène ou l'analogue d'haptène ou la liaison du conjugué d'haptène avec l'élément spécifique immobilisé de liaison, selon les circonstances, par l'intermédiaire de la réflection globale frustrée.

8. Méthode selon la revendication 7, dont la phase a1) comporte les phases suivantes
i) addition de l'élément spécifique de liaison à l'haptène ou l'analogue,
ii) l'élution pour éliminer la matière non reliée, et
iii) la mise en contact de l'éprouvette avec la couche de matière diélectrique transparente, sur laquelle l'élément spécifique de liaison est reliée de façon réversible avec l'haptène ou l'analogue en immobilisation.

9. Méthode selon la revendication 7, dont l'élément spécifique immobilisé de liaison de la phase a2) est un anticorps de l'haptène ou de l'analogue d'haptène, ou d'un de leurs fragments.

10. Méthode selon la revendication 7, dont la phase a2) comporte les phases suivantes:
i) liaison irréversible du conjuguée avec l'élément spécifique de liaison immobilisé de liaison, et
ii) la mise en contact de l'éprouvette avec la surface portant l'espèce chimique immobilisé.

11. Méthode selon la revendication 7, dont l'haptène ou l'analogue d'haptène à la phase a2) se conjugue avec une espèce chimique sous forme d'élément de liaison spécifique d'un autre réactif qui ne réagit pas avec l'haptène ou l'analogue d'haptène, ni avec l'élément de liaison spécifique immobilisé de l'haptène ou l'analogue d'haptène, la méthode comportant en phase supplémentaire l'addition de l'autre réactif.

12. Méthode selon la revendication 11, dont le réactif supplémentaire est un conjugué de l'associé spécifique de liaison avec l'espèce chimique auquel l'haptène est conjugué.

13. Méthode selon la revendication 12, dont le réactif supplémentaire est un conjugué de l'associé spécifique de liaison avec une enzyme apte à catalyser la production d'un produit de réaction captable situé en surface ou à sa proximité.
